# FASCICULE DE BREVET EUROPEEN

(11) **EP 2 300 399 B1**
(45) Date de publication et mention de la délivrance du brevet: **22.04.2020**
(21) Numéro de dépôt: 09794056.3
(22) Date de dépôt: 09.07.2009
(51) Int. Cl.: C07C 21/18, C07C 1/24, C07C 41/09, C07C 11/02, C07C 17/10, C07C 17/16, C07C 17/087, C07C 17/25, C07C 17/275

(54) **HYDRO(CHLORO)FLUOROOLEFINES ET LEUR PROCÉDÉ DE PRÉPARATION**
HYDRO(CHLOR)FLUOROLEFINE UND VERFAHREN ZU IHRER HERSTELLUNG
HYDRO(CHLORO)FLUOROOLEFINS AND METHOD FOR PREPARATION THEREOF

(30) Priorité: 10.07.2008 FR 0803941
(43) Date de publication de la demande: 30.03.2011
(73) Titulaire: ARKEMA FRANCE, 92700 Colombes (FR)
(72) Inventeur: DUBOIS, Jean-Luc, 69390 Millery (FR)
(74) Mandataire: Hirsch & Associés
(86) Numéro de dépôt international: PCT/IB2009/006199
(87) Numéro de publication internationale: WO 2010/004415

(56) Documents cités:
- EP-A1- 1 770 081
- EP-A1- 1 953 129
- WO-A1-2005/077865
- WO-A1-2005/108334
- WO-A1-2007/055361
- WO-A2-2008/040969
- WO-A2-2008/067627
- DE-A1- 4 438 902
- FR-A1- 2 147 207
- US-A- 4 873 392
- US-B1- 6 369 284
- F. BOBERG, R. VOSS: "Markierte Verbindungen, XXVI Das 1,2,3-Trichlorpropenylium-Ion als Intermediat bei der Fluorierung von 1,2,3,3-Tetrachlor-1-propen" ZEITSCHRIFT FÜR NATURFORSCHUNG, TEIL B: ANORGANISCHE CHEMIE, ORGANISCHE CHEMIE, vol. 31B, no. 5, 1976, pages 583-585, XP008101302
- HADDACH M ET AL: "Synthesis of [2-14C]Perfluorohexane" JOURNAL OF LABELLED COMPOUNDS AND RADIOPHARMACEUTICALS, JOHN WILEY, CHICHESTER, GB, vol. 42, 1 janvier 1999 (1999-01-01), pages 227-231, XP002337936 ISSN: 0362-4803

## Description

### DOMAINE DE L'INVENTION

L'invention a pour objet un procédé de préparation d' hydro(chloro)-fluorooléfines , à partir de matières premières renouvelables.

### ARRIERE-PLAN TECHNOLOGIQUE

Les hydro(chloro)fluorooléfines ou H(C)FOs sont connues notamment pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. A la différence des CFC et des HCFC, qui sont potentiellement dangereux pour la couche d'ozone, les HFOs ne contiennent pas de chlore et donc ne posent pas de problème pour la couche d'ozone.

Le document WO2008/040969 décrit un procédé de production de 1234yf, ainsi qu'une étape d'addition de tétrachlorométhane sur l'éthylène.

Ces composés sont synthétisés à partir des composés hydrocarbonés classiques, à savoir issus de l'industrie du pétrole. Si les HFOs ne sont pas dangereux pour la couche d'ozone, le bilan écologique de leur production n'est pas parfait et notamment le bilan CO₂. Ces composés ont remplacé les HFC du fait d'un potentiel GWP (Global Warming Potential) plus faible. Cependant, les procédés actuels de fabrication des H(C)FOs contribuent encore au réchauffement climatique.

L'invention a donc pour objet de réduire le réchauffement de la planète lors de la fabrication de H(C)FOs, en réduisant les émissions de gaz à effet de serre liées à leur fabrication.

L'invention a donc pour objet d'améliorer l'empreinte carbone (émissions de gaz à effet de serre cumulées liées aux productions de matières première et au procédé de production de H(C)FO).

### RESUME DE L'INVENTION

L'invention a pour objet un procédé de préparation d'une composition de composés provenant au moins en partie de biomateriau, les composés étant choisis dans le groupe consistant en 2,3,3,3-tétrafluoro-1-propène (1234yf) et 3,3,3-trifluoro-2-chloro-1-propène (1233xf), comprenant l'étape de fourniture d'une ou plusieurs chaînes carbonées comprenant un ou plusieurs atomes de carbone ayant une teneur en biocarbone d'au moins 1%, déterminée en application des normes ASTM D6866 et ASTM D 7026, et la transformation par synthèse en le composé halogéné recherché.

Selon l'invention, l'étape de fourniture comprend une étape de production d'alcool, notamment de méthanol, éthanol, n-propanol et n-butanol.

Selon l'invention, l'étape de production d'alcool se fait par fermentation de biomasse.

Selon l'invention, l'étape de production d'alcool comprend les sous-étapes suivantes: (i) production de méthane à partir de biomasse, (ii) réformage à la vapeur de celui-ci en un gaz de synthèse ou production de gaz de synthèse par gazéification directe de biomasse et (iii) production d'alcool à partir de ce gaz de synthèse.

Selon l'invention, l'étape de production d'alcool peut comprendre les sous-étapes suivantes: (i) production de méthane à partir de biomasse, (ii) oxydation directe en méthanol.

Selon l'invention, l'alcool est transformé en composé halogéné saturé.

Selon un mode de réalisation, le méthanol est converti en composé CHₘClₙF₄₋ₘ₋ₙ avec m qui peut représenter chacun un entier de 0 à 3 et n qui peut représenter chacun un entier de 0 à 4 et m+n est au plus égal à 4.

Selon un mode de réalisation, le composé CHₘClₙF₄₋ₘ₋ₙ réagit avec au moins une oléfine.

L'oléfine peut être obtenue par transformation de l'alcool ou par transformation du méthanol qui est ensuite déshydraté en oléfine.

L'étape de synthèse comprend au moins une étape de chloration suivie d'au moins une étape de fluoration partielle ou totale.

Selon un mode de réalisation, l'étape de synthèse comprend au moins une étape de déhydrohalogénation.

### DESCRIPTION DETAILLEE DE MODES DE REALISATION

L'invention utilise les produits d'origine naturelle comme produits de départ. Le carbone d'un biomatériau provient de la photosynthèse des plantes et donc du CO₂ atmosphérique. La dégradation (par dégradation, on entendra aussi la combustion/incinération en fin de vie) de ces matériaux en CO₂ ne contribue donc pas au réchauffement puisqu'il n'y a pas d'augmentation du carbone émis dans l'atmosphère. Le bilan CO₂ des biomatériaux est donc nettement meilleur et contribue à réduire l'empreinte carbone des produits obtenus (seule l'énergie pour la fabrication est à prendre en compte). Au contraire, un matériau d'origine fossile se dégradant aussi en CO₂ va contribuer à l'augmentation du taux de CO₂ et donc au réchauffement climatique.

Les composés préparés selon le procédé de l'invention auront donc une empreinte carbone qui sera meilleure que celle de composés obtenus à partir d'une source fossile.

Le procédé selon l'invention améliore donc aussi le bilan écologique lors de la fabrication des H(C)FOs.

Le terme de "bio-carbone" indique que le carbone est d'origine naturelle et provient d'un biomatériau, comme indiqué ci-après. La teneur en biocarbone et la teneur en biomatériau sont des expressions désignant la même valeur.

Un matériau d'origine renouvelable ou biomatériau est un matériau organique dans lequel le carbone provient de CO₂ fixé récemment (à l'échelle humaine) par photosynthèse à partir de l'atmosphère. Sur terre, ce CO₂ est capté ou fixé par les plantes. En mer, le CO₂ est capté ou fixé par des bactéries ou des végétaux ou algues microscopiques procédant à une photosynthèse. Un biomatériau (100% de carbone origine naturelle) présente un ratio isotopique ¹⁴C/¹²C supérieur à 10⁻¹², typiquement de l'ordre de 1,2 x 10⁻¹², tandis qu'un matériau fossile a un ratio nul. En effet, l'isotope ¹⁴C se forme dans l'atmosphère et est ensuite intégré par photosynthèse, selon une échelle de temps de quelques dizaines d'années au plus. La demi-vie du ¹⁴C est de 5730 ans. Donc les matériaux issus de la photosynthèse, à savoir les végétaux de manière générale, ont nécessairement une teneur maximale en isotope ¹⁴C.

La détermination de la teneur en biomatériau ou teneur en biocarbone est déterminée en application des normes ASTM D 6866 (ASTM D 6866-06) et ASTM D 7026 (ASTM D 7026-04). La norme ASTM D 6866 a pour objet *"Determining the Biobased Content of Natural Range Materials Using Radiocarbon and Isotope Ratio Mass Spectrometry Analysis",* tandis que la norme ASTM D 7026 a pour objet *"Sampling and Reporting of Results for Détermination of Biobased Content of Materials via Carbon Isotope Analysis".* La seconde norme renvoie dans son premier paragraphe à la première.

La première norme décrit un test de mesure du ratio ¹⁴C/¹²C d'un échantillon et le compare avec le ratio ¹⁴C/¹²C d'un échantillon référence d'origine 100% renouvelable, pour donner un pourcentage relatif de C d'origine renouvelable dans l'échantillon. La norme est basée sur les mêmes concepts que la datation au ¹⁴C, mais sans faire application des équations de datation.

Le ratio ainsi calculé est désigné comme le "pMC" (percent Modem Carbon). Si le matériau à analyser est un mélange de biomatériau et de matériau fossile (sans isotope radioactif), alors la valeur de pMC obtenu est directement corrélée à la quantité de biomatériau présent dans l'échantillon. La valeur de référence utilisée pour la datation au ¹⁴C est une valeur datant des années 1950. Cette année a été choisie en raison de l'existence d'essais nucléaires dans l'atmosphère qui ont introduit des grandes quantités d'isotopes dans l'atmosphère après cette date. La référence 1950 correspond à une valeur pMC de 100. Compte tenu des essais thermonucléaires, la valeur actuelle à retenir est d'environ 107.5 (ce qui correspond à un facteur de correction de 0.93). La signature en carbone radioactif d'un végétal actuel est donc de 107.5. Une signature de 54 pMC et de 99 pMC correspondent donc à une quantité de biomatériau dans l'échantillon de 50% et de 93%, respectivement.

La norme ASTM D 6866 propose trois techniques de mesure de la teneur en isotope ¹⁴C:
- LSC (Liquid Scintillation Counting) spectrométrie à scintillation liquide. Cette technique consiste à compter des particules "Bêta" issues de la désintégration du ¹⁴C. On mesure le rayonnement Bêta issu d'un échantillon de masse connue (nombre d'atomes C connu) pendant un certain temps. Cette "radioactivité" est proportionnelle au nombre d'atomes de ¹⁴C, que l'on peut ainsi déterminer. Le ¹⁴C présent dans l'échantillon émet des rayonnements β, qui au contact du liquide scintillant (scintillateur) donnent naissance à des photons. Ces photons ont des énergies différentes (comprises entre 0 et 156 keV) et forment ce que l'on appelle un spectre de ¹⁴C. Selon deux variantes de cette méthode, l'analyse porte soit sur le CO₂ préalablement produit par l'échantillon carboné dans une solution absorbante appropriée, soit sur le benzène après conversion préalable de l'échantillon carboné en benzène. La norme ASTM D 6866 donne donc deux méthodes A et C, basées sur cette méthode LSC.
- AMS/IRMS (Accelerated Mass Spectrometry couplé avec Isotope Radio Mass Spectrometry). Cette technique se base sur la spectrométrie de masse. L'échantillon est réduit en graphite ou en CO₂ gazeux, analysé dans un spectromètre de masse. Cette technique utilise un accélérateur et un spectromètre de masse pour séparer les ions ¹⁴C des ¹²C et donc déterminer le rapport des deux isotopes.

La composition de composés préparés selon le procédé de l'invention proviennent au moins en partie de biomatériau et présentent donc une teneur en biomatériau d'au moins 1%. Cette teneur est avantageusement plus élevée, notamment jusqu'à 100%. Les composés selon l'invention peuvent donc comprendre 100% de bio-carbone ou au contraire résulter d'un mélange avec une origine fossile.

Selon un mode de réalisation, le ratio isotopique ¹⁴C/¹²C est compris entre 0,2 x 10⁻¹² et 1,2 x 10⁻¹².

Les composés préparés selon le procédé de l'invention sont, comme indiqué supra, des hydro(chloro)-fluorooléfines choisies dans le groupe consistant en 2,3,3,3-tétrafluoro-1-propène (1234yf) et 3,3,3-trifluoro-2-chloro-1-propène (1233xf),

Pour produire les H(C)FOs à base de biocarbone, on produit dans un premier temps un composé carboné non halogéné. Dans un second temps, ce composé non halogéné est soumis à des réactions de chloration puis de fluoration ou de fluoration directe (et éventuellement de chloration). Des réactions de couplage, pyrolyse, élimination, addition sont aussi possibles. On obtient ainsi la composition de H(C)FOs du procédé de l'invention.

A titre d'exemple de réactions susceptibles de produire des composés non halogénés à base de biocarbone, on peut citer les réactions suivantes.

La production de méthane à partir de la biomasse est connue. Le méthane du biogas résulte de la méthanisation ou digestion anaérobie de déchets fermentescibles. Les sources courantes sont les décharges, la collecte sélective des déchets putrescibles (éventuellement par l'usage de digesteurs), les boues de station d'épuration, les effluents d'élevage, les effluents des industries alimentaires, ou à partir d'un lac (par exemple le lac Kivu), etc. Le biogas contient une proportion majoritaire de méthane.

Ce méthane subit ensuite une réaction de réformage à la vapeur ou SMR (Steam Methane Reforming). A l'issue de cette réaction on obtient un mélange de CO et d'hydrogène selon un ratio variable (typiquement environ 2 à 3), aussi appelé gaz de synthèse ou syngas. Ce syngas est utilisé par exemple pour la production d'hydrocarbures par la réaction de Fischer-Tropsch, hydrocarbures qui peuvent ensuite être transformés en des produits divers notamment oléfines par des réactions de valorisation classiques, dites upgrading en anglais. Ce syngas peut aussi, en fonction du ratio H₂:CO, et/ou en fonction des catalyseurs utilisés, être transformé en méthanol ou en alcools supérieurs.

On peut aussi prévoir la gazéification directe de biomasse en gaz de synthèse ou syngas, par réaction du carbone de la biomasse en présence par exemple d'oxygène (pur ou de l'air) ou d'eau, à une température plus ou moins élevée (par exemple de 800 à 1000°C) à une pression par exemple voisine de la pression atmosphérique.

Sur la transformation du syngas en méthanol ou en alcool supérieur, on peut se référer à "Procédés de pétrochimie, IFP, ENSPM", 1985, 2e édition, pp 90-104 et à "Fundamentals of Industrial Catalytic Processes", Wiley, 2e édition, 6.4.8.

Il est aussi possible de procéder à une oxydation directe (contrôlée) du méthane directement en méthanol.

L'éthanol ou un alcool supérieur comme le propanol ou le butanol peut aussi être produit directement à partir de la biomasse. On fermente une biomasse à l'aide d'une levure (par exemple Saccharomyces Cerevisiae) ou une bactérie (par exemple Zymomonas ou Clostrodium). De tels procédés sont connus de l'homme du métier. La biomasse de départ est variable, notamment en fonction du microorganisme utilisé, et elle peut être éventuellement hydrolysée en des oses élémentaires.

D'autres méthodes comme celle dans laquelle le méthanol obtenu à partir de la biomasse est ensuite être converti en diméthyléther (DME) pourraient être aussi utilisées. Le DME est ensuite déshydraté pour conduire à des oléfines inférieures, typiquement éthylène et propylène. Cette réaction est classique et bien connue de l'homme du métier. On pourra se référer aux documents suivants: "Heterogeneous catalysts in Industrial Practice", Charles N. Satterfield, Second Edition, McGraw-Hill Inc, 7.7.9 Methanol to gasoline, p.255; UOP "UOP/HYDRO MTO Process Methanol to Olefins Conversion", 2007, UOP 4522-3 UOP-PTE 0708-002; Lurgi, "Methanol-To-Propylene - MTP®", brochure commerciale, pp 1-4.

Selon encore une autre méthode, les alcools supérieurs comme l'éthanol, le propanol et le butanol pourraient ensuite être déshydratés de façon classique pour obtenir des oléfines par élimination d'eau.

La biomasse de départ peut être une biomasse lignocellulosique (bois, canne à sucre, paille, etc.) ou une biomasse à glucides (céréales, betterave, etc.) facilement hydrolysable.

Les oléfines ainsi obtenues pourraient ensuite être converties par des réactions de fluoration et/ou de chloration connues de l'homme du métier.

Par exemple, à partir de l'éthylène, on peut obtenir le chlorure de vinyle et dichloroéthane par chloration (voir Procédé CVM Chlorure de Vinyle Monomère de J.C LANET). A partir d'éthylène, on peut aussi mettre en oeuvre le procédé comprenant la réaction de tétrachlorométhane sur l'éthylène pour conduire au 1,1,1,3-tétrachloropropane. Ce dérivé peut ensuite être converti en le 3,3,3-trifluoro-1-propène par des réactions de fluoration (éventuellement en présence d'un catalyseur) et déhydrohalogénation. Le 3,3,3-trifluoro-1-propène peut encore être converti par chloration avec Cl₂ en 1,1,1-trifluoro-2,3-dichloro-propane. Ce dernier composé peut encore subir une réaction de déshydrochloration en 3,3,3-trifluoro-2-chloro-1-propène, qui peut ensuite être mis à réagir avec HF pour conduire au 3,3,3,2-tétrafluoro-2-chloro-propane. Ce composé peut ensuite être soumis à une réaction de déshydrochloration pour conduire au 2,3,3,3-tétrafluoro-1-propène. Le 3,3,3-trifluoro-2-chloro-1-propène peut aussi être obtenu à partir du dérivé chloré 1,1,2,3-tétrachloro-1-propène (1230xa) .

L'éthylène peut aussi subir une réaction de fluoration pour conduire au dérivé monofluoroéthylène ou difluoroéthylène ou encore d'autres dérivés fluorés.

Le monofluoroéthylène peut réagir avec CF₃Cl pour conduire au composé de formule CF₃CH₂CHFCl, qui est ensuite déhydrohalogéné (par ex. sur potasse) en 1,3,3,3-tétrafluoro-1-propène.

Le difluoroéthylène peut réagir avec un composé CHFCl₂, pour produire un composé de formule CHClFCH₂CClF₂, qui peut ensuite être exposé à des conditions de déhydrohalogénation et/ou fluoration pour conduire au composé 1,3,3,3-tétrafluoro-1-propène.

A partir de propylène, on peut citer la réaction comprenant la réaction avec du chlore pour produire le dérivé CCl₃CHClCH₂Cl (catalyseur Au/TiO2), qui est fluoré en CF₃CHClCH₂F, qui est ensuite déhydrochloré pour conduire au composé 1,3,3,3-tétrafluoro-1-propène.

Le 2,3,3,3-tétrafluoro-1-propène peut être préparé par déhydrofluoration à partir de 1,1,1,2,3-pentafluoropropane ou par déhydrochloration à partir de 1,1,1,3-tétrafluoro-2-chloro-propane.

Le 2,3,3,3-tétrafluoro-1-propène peut aussi être préparé par déhydrofluoration à partir de 1,1,1,2,2-pentafluoropropane (ce dernier composé peut être obtenu à partir du composé pentachloré correspondant via le trichloroacétone ou par des étapes de chloration et fluoration successives via l'intermédiaire 2,2-difluoropropane.

On peut citer encore les réactions visées dans le document Knunyants et al, Journal of the USSR Academy of Sciences, Chemistry Department, "reactions of fluoro-olefins", report 13., "catalytic hydrogenation of perfluoro-olefins", 1960.

De façon générale, on peut procéder à une fluoration et/ou une chloration des oléfines, conduisant éventuellement à un composé saturé, qui peut subir des réactions de déshydrohalogénation pour conduire à l'oléfine recherchée.

Il est possible de préparer des composés chlorés à partir du méthanol, par réaction de HCl sur du méthanol, ce qui conduit à du monochlorométhane et de l'eau. Le monochlorométhane peut ensuite être à son tour converti par action de Cl₂, jusqu'au tétrachlorométhane. Le tétrachlorométhane est un intermédiaire connu dans la chimie des oléfines halogénées. Les dérivés chlorés du méthane peuvent être fluorés à l'aide d'acide fluorhydrique, pour donner des dérivés de méthane plus ou moins fluorés (contenant ou non encore des atomes de chlore). Ces composés de méthane chlorés et/ou fluorés peuvent être soumis à des réactions de couplage par exemple ou d'addition.

## Revendications

1. Procédé de préparation d'une composition de composés provenant au moins en partie de biomatériau les composés étant choisi dans le groupe consistant en 2,3,3,3-tétrafluoro-1-propène (1234yf) ou 3,3,3-trifluoro-2-chloro-1-propène (1233xf), comprenant l'étape de fourniture d'une ou plusieurs chaînes carbonées comprenant un ou plusieurs atomes de carbone ayant une teneur en biocarbone d'au moins 1%, déterminée en application des normes ASTM D6866 et ASTM D 7026, et la transformation par synthèse en le composé halogéné recherché, **caractérisée en ce que**
l'étape de fourniture comprend une étape de production d'alcool par fermentation de biomasse; ou bien l'étape de fourniture comprend une étape de production d'alcool selon les sous-étapes suivantes: (i) production de méthane à partir de biomasse, (ii) réformage à la vapeur de celui-ci en un gaz de synthèse, puis transformation du gaz de synthèse en alcool; ou bien
l'étape de fourniture comprend une étape de production d'alcool par gazéification directe de biomasse en un gaz de synthèse, puis transformation du gaz de synthèse en alcool, et **caractérisé en ce que** l'étape de synthèse en le composé halogéné comprend au moins une étape de chloration suivie d'au moins une étape de fluoration partielle ou totale et **caractérisé en ce que** l'alcool est transformé en composé halogéné saturé.

2. Procédé selon la revendication 1, dans lequel l'étape de fourniture comprend une étape de production d'alcool, parmi le méthanol, éthanol, n-propanol et n-butanol.

3. Procédé selon la revendication 2, dans lequel l'étape de production d'alcool comprend les sous-étapes suivantes: (i) production de méthane à partir de biomasse, (ii) oxydation directe en méthanol.

4. Procédé selon la revendication 1, dans lequel le méthanol est converti en composé CHₘClₙF₄₋ₘ₋ₙ avec m qui peut représenter chacun un entier de 0 à 3 et n qui peut représenter chacun un entier de 0 à 4 et m+n est au plus égal à 4.

5. Procédé selon la revendication 4, dans lequel le composé CHₘClₙF₄₋ₘ₋ₙ réagit avec au moins une oléfine.

6. Procédé selon l'une des revendications 1 à 5, dans lequel l'étape de synthèse comprend au moins une étape de déhydrohalogénation.

## Patentansprüche

1. Verfahren zum Herstellen einer Zusammensetzung von Verbindungen, die mindestens teilweise aus Biomaterial stammen, wobei die Verbindungen ausgewählt sind aus der Gruppe bestehend aus 2,3,3,3-Tetrafluor-1-propen (1234yf) oder 3,3,3-Trifluor-2-chlor-1-propen (1233xf), umfassend den Schritt zum Bereitstellen einer oder mehrerer Kohlenstoffketten, umfassend ein oder mehrere Kohlenstoffatome, die einen Gehalt an Biokohlenstoff von mindestens 1 % aufweisen, der unter Anwendung der Normen ASTM D6866 und ASTM D7026 bestimmt wurde, und die Umwandlung durch Synthese in die gewünschte halogenierte Verbindung,
**dadurch gekennzeichnet, dass**
- der Schritt zum Bereitstellen einen Schritt zum Herstellen von Alkohol durch Fermentation von Biomasse umfasst;
oder
- der Schritt zum Bereitstellen auch einen Schritt zum Herstellen von Alkohol gemäß den folgenden Teilschritten umfasst:
(i) Herstellen von Methan aus Biomasse,
(ii) Dampfreformieren desselben in ein Synthesegas, dann Umwandeln des Synthesegases in Alkohol;
oder
der Schritt zum Bereitstellen auch einen Schritt zum Herstellen von Alkohol durch direkte Vergasung von Biomasse in ein Synthesegas, dann Umwandeln des
Synthesegases in Alkohol umfasst,
und **dadurch gekennzeichnet, dass** der Syntheseschritt in die halogenierte Verbindung mindestens einen Schritt zum Chlorieren umfasst, gefolgt von mindestens einem Schritt zum teilweisen oder vollständigen Fluorieren, und **dadurch gekennzeichnet, dass** der Alkohol in eine gesättigte halogenierte Verbindung umgewandelt wird.

2. Verfahren nach Anspruch 1, wobei der Schritt zum Bereitstellen einen Schritt zum Herstellen von Alkohol wie Methanol, Ethanol, n-Propanol und n-Butanol, umfasst.

3. Verfahren nach Anspruch 2, wobei der Schritt zum Herstellen von Alkohol die folgenden Teilschritte umfasst:
(i) Herstellen von Methan aus Biomasse,
(ii) Direktoxidation zu Methanol.

4. Verfahren nach Anspruch 1, wobei das Methanol in die Verbindung CHₘClₙF₄₋ₘ₋ₙ umgewandelt wird, wobei m für jeweils eine ganze Zahl von 0 bis 3 stehen kann, und n für jeweils eine ganze Zahl von 0 bis 4 stehen kann und m+n höchstens gleich 4 ist.

5. Verfahren nach Anspruch 4, wobei die Verbindung CHₘClₙF₄₋ₘ₋ₙ mit mindestens einem Olefin umgesetzt wird.

6. Verfahren nach einem der Ansprüche 1 bis 5, wobei der Schritt zum Synthetisieren mindestens einen Schritt zum Dehydrohalogenieren umfasst.

## Claims

1. Process for the preparation of a composition of compounds originating at least partly from biomaterials, the compounds being selected from the group consisting of 2, 3, 3, 3-tetrafluoro-1-propene (1234yf) or 3, 3, 3-trifluoro-2-chloro-1-1-propene (1233xf), comprising the step of providing one or more carbon chains comprising one or more carbon atoms having a biocarbon content of at least 1%, determined according to ASTM D6866 and ASTM D 7026, and converting by synthesis into the desired halogen compound, **characterized in that** the supply step includes a step of alcohol production by fermentation of biomass ; or the supply step includes a production step; or of alcohol according to the following sub-steps: (i) production of methane from biomass, (i) steam reforming thereof into a synthesis gas and then converting the synthesis gas into alcohol; or the supply step comprises a step of production of alcohol by direct gasification of biomass into a synthesis gas, followed by conversion of the synthesis gas into alcohol, and **characterized in that** the step of synthesis into the halogen compound comprises at least one chlorination step followed by at least one step of partial or complete fluorination and **characterized in that** the alcohol is converted into a saturated halogen compound.

2. A process according to claim 1, wherein the providing step comprises a step of producing an alcohol from among methanol, ethanol, n-propanol and n-butanol.

3. A process according to claim 2, wherein the alcohol production step comprises the following substeps: (i) production of methane from biomass, (i) direct oxidation to methanol.

4. A process according to claim 1, wherein methanol is converted into the compound CHₘClₙF₄₋ₘ₋ₙ with m which may each represent an integer from 0 to 3 and n which may each represent an integer from 0 to 4 and m+n is at most 4.

5. A process according to claim 4, wherein the compound CHₘClₙF₄₋ₘ₋ₙ is reacted with at least one olefin.

6. A process according to one of claims 1 to 5, wherein the synthesis step comprises at least one dehydrohalogenation step.
